# EUROPEAN PATENT SPECIFICATION

(11) **EP 2 910 178 B1**
(45) Date of publication and mention of the grant of the patent: **27.12.2017**
(21) Application number: 15155558.8
(22) Date of filing: 18.02.2015
(51) Int. Cl.: A61B 1/00, A61B 1/04, A61B 34/00, A61B 90/00

(54) **GASTROINTESTINAL TRACT DIAGNOSIS SYSTEM AND CONTROL METHOD FOR THE SAME**
MAGEN-DARM-TRAKT-DIAGNOSESYSTEM UND STEUERVERFAHREN DAFÜR
SYSTÈME DE DIAGNOSTIC DU TRACTUS GASTRO-INTESTINAL ET SON PROCÉDÉ DE COMMANDE

(30) Priority: 19.02.2014 TW 103105479
(43) Date of publication of application: 26.08.2015
(73) Proprietor: INSIGHT MEDICAL SOLUTIONS INC., Hsinchu City (TW)
(72) Inventor: Hong, Hei Tai, 308 Baoshan Township, Hsinchu County (TW); Tsai, Huai- Fang, Zhubei City, Hsinchu County (TW); Lu, Shih Chieh, Hsinchu City (TW); Wu, Sing, 320 Zhongli City, Taoyuan County (TW); Sung, Tze-Yun, Taoyuan City (TW); Tsai, Ping Chun, Hsinchu City (TW)
(74) Representative: Viering, Jentschura & Partner mbB Patent- und Rechtsanwälte

(56) References cited:
- EP-A1- 2 087 832
- EP-A1- 2 116 169
- EP-A2- 2 135 545

## Description

### BACKGROUND OF THE INSTANT DISCLOSURE

### Field of the Invention

The instant disclosure is related to a gastrointestinal tract diagnosis system and control method for the same, in particular, a system and control method for the same which are related to utilizing a predetermined magnetic force formed from an exterior magnetic field as well as to having a feedback mechanism of magnetic force control for controlling the desired viewing direction and stabilizing the predetermined magnetic force.

### Description of Related Art

EP 2 087 832 A1 discloses a position detection system according to the preamble of claim 1.

EP 2 135 545 A2 discloses a magnetically guiding system including: a capsule medical device that has a magnet provided therein; an information acquiring unit that acquires physical information about magnetic guiding of the capsule medical device; a magnetic field generating unit, that generates a magnetic field for magnetically guiding the capsule medical device; and a control unit that sets a magnetic field condition based on the physical information acquired by the information acquiring unit and controls the magnetic field generating unit to generate a magnetic field corresponding to the magnetic field condition.

EP 2 116 169 A1 discloses 3 degrees-of-freedom motion of a capsule medical device inserted into a subject. It operates a capsule endoscope with 6 degrees-of-freedom motion by using a magnetic field generator with respect to the capsule endoscope inserted into the subject. The operating device comprises an operating unit including a fixed unit and a movable unit. The operating unit has a three-dimensional shape substantially identical to the capsule endoscope and is a holdable size. The force sensor detects force information of the movable unit when the movable unit of the operating unit is operated once or continuously. The force information detected by the force sensor is output as instruction information for instructing 6 degrees-of-freedom motion of the capsule endoscope.

One of the most commonly known gastrointestinal tract check devices is related to a device having a flexible hard tube connected with an endoscope that is able to be inserted inside a body from the mouth of a person who is receiving the check in a way like sword swallowing, and the device can be inserted deeper to reach and check the gastrointestinal tract. However, the check methods based on such devices with flexible hard tubes often cause discomfort, disgorging or fear by the check receivers due to the foreign invasion to the gastrointestinal tract, even resulting in failing to finish a complete check. If the above method is found not fit for a check recipient due to the above reasons, anesthesia can be employed to overcome the above problems, but anesthesia itself may still bring potential risks and bad effects to the health of the check recipient.

Thereafter, as is shown in the prior art of U.S. Pat. No. 5604531, an in vivo video camera system putting a camera into a swallowable capsule for check recipients has been developed. When the swallowable capsule-type camera is swallowed by check recipients, the capsule-type camera can be continuously pushed to the tail end of the gastrointestinal tract by the process of the peristalsis motions of the gastrointestinal tract. Photographing the gastrointestinal tract of check recipients can be done during this process. The capsule-type camera can be excreted by the check receiver in 8 hours or so and be retaken by medical staffs to obtain the image data therein for determination. But the capsule-type camera shows its value only to the small intestine where a disease occurring possibility is lower and cannot meet the needs for the check of the stomach where disease occurring possibility is higher than the small intestine. Besides, the check of the large intestine can be done by the colonoscopy instead of the above capsule-type camera. Moreover, the capsule-type camera still has the shortcoming of a big potential risk of being unable to be successfully excreted by the check receiver. If the situation does occur, an operation will inevitably have to be performed to get the capsule-type camera out, resulting in more unnecessary burden and waste of medical resources. The capsule-type camera also has the defect of being unable to be retaken any time in an active manner by the medical staff and can only be retaken passively after being excreted out of anus by check recipients who swallowed the capsule-type camera. Hence, the time length for checking cannot be decided by medical staff. Once any other medical requirements or accidents happen to require immediate remove of the capsule-type camera, the capsule-type camera will not be able to be removed by non-surgical operation. In addition, the capsule-type camera does not record real-time images, and it almost takes one day or so for a check recipient to excrete the camera since the time the camera is swallowed. After being excreted, it often takes two to three days or longer for a medical stuff to obtain, record, file, check and determine the image therein, because the capsule-type camera cannot be used to find the nidus in an active manner of manual operation, therefore complete images from the beginning to the end of the intestine are recorded by the above capsule-type endoscope to prevent any possible missing of recording, resulting in a great deal of redundant image data. The medical staff spends much time searching the image data to really capture the nidus from the great amount of image data. Also, the capsule-type endoscope unnecessarily consumes a lot of electrical power due to excessive performing of image shooting.

Furthermore, there is prior art related to in vivo controlling of an endoscope by means of a distal magnetic force. But a shaking state of the endoscope results instead of maintaining a stabilized state of the endoscope. Clear desired images or observation of the target distinctly is unavailable. To improve the problem, more powerful magnetic force is applied to perfect the controlling of the endoscope. However, over-friction by the endoscope may hurt the inner wall of the body, such as wall of the stomach.

### SUMMARY OF THE INVENTION

The object of the instant disclosure is to provide a gastrointestinal tract diagnosis system to improve the movement and control the problem of the endoscope inside a cavity and render the surveillance of the gastrointestinal tract to be carried out under a more stabilized state, so as to achieve a more distinct surveillance and image capture.
The aforementioned object is achieved by a gastrointestinal tract diagnosis system according to claim 1 and a control method of a gastrointestinal tract diagnosis system according to claim 11.

To achieve the aforementioned object, the instant disclosure provide a gastrointestinal tract diagnosis system, at least comprising: an endoscope having a magnetic plate therein, the magnetic plate having a first magnetic pole and a second magnetic pole respectively distributed at a lower half portion and an upper half portion defined along a thickness direction; and a magnetic control device including: a magnetic stick including a first electric magnetic pole at a terminal portion of the magnetic stick and a second magnetic pole located at a base portion of the magnetic stick; a magnetic force output module for being used to output a predetermined current to the magnetic stick so as to allow the magnetic stick to generate a predetermined current generating a predetermined magnetic force for interacting with the first magnetic pole and the second magnetic pole; wherein the predetermined magnetic force is adapted to draw the magnetic plate to control the movement of the endoscope, and the magnetic plate is adapted to cause a feedback magnetic force to a sensor of the magnetic stick, converting the predetermined current into an offset current, and that the magnetic control device further includes a magnetic force feedback module, wherein the magnetic force feedback module is adapted to calibrate the offset current, resuming the offset current back to the predetermined current, so as to stabilize the predetermined magnetic force.

In addition, to achieve the aforementioned object, the instant disclosure provides a control method of a gastrointestinal tract diagnosis system, at least comprising the following steps: (a) providing an endoscope having a transmission line and a magnetic plate, and delivering the endoscope into a cavity body with a first section of the transmission line and a second section of the transmission line being left outside of the cavity body; (b) providing a magnetic device at least having a magnetic stick and a magnetic force output module, and activating the magnetic device to allow the magnetic force output module to generate a predetermined magnetic force to electro-magnetically attract the magnetic plate, so as to observe the cavity body by directing the endoscope; (c) adjusting movement, rotation and view field of the endoscope inside the cavity body by means of the magnetic device to take photographs internally of the cavity body; (d) repeating step (b) to step (c) for a number of times till the endoscope finishes taking photographs inside the cavity body; (e) terminating the magnetic device to cease the predetermined magnetic force; and (f) pulling the second section of the transmission line to render the endoscope to move to the outside of the cavity body from inside cavity body, so as to retake the endoscope, further comprising a step as the step (b) is progressed to the step (c): providing a magnetic force feedback module (23), and keeping surveillance of a feedback magnetic force generated by the magnetic plate (11) upon the magnetic stick (21), the feedback magnetic force rendering the predetermined magnetic force becoming an offset magnetic force, the magnetic force feedback module (23) sending out a calibration command to the magnetic force output module (22) in accordance with the feedback magnetic force, allowing the offset magnetic force to be rectified back to the predetermined magnetic force.

In summary, the movement of the endoscope can be better controlled, resulting in the effect of more distinct image capture and surveillance. The injury to the cavity body resulting from the endoscope due to excessive attraction force can also be prevented. In particular, the improper physical friction as well as hits the gastrointestinal tract or stomach may suffer can be greatly reduced. When the endoscope is controlled under a proper magnetic attraction force, the nidus can be found precisely, and image capturing can be done immediately so that any unnecessary picture-shooting, power consumption, as well as the huge time and manual labor power required for massive picture-screening can all be greatly decreased. The control method derived from the system of the instant disclosure particularly includes steps of using transmission lines of which the line diameter conforms to the diameter of the general esophageal tube and of which the material is of biocompatible soft elements. Discomfort resulting from the endoscopes utilizing hard tubes will not be caused, and the endoscope of the instant disclosure can also be taken back easier than the known capsule-endoscopes can be.

Advantages and the essence of the instant disclosure can be further understood by the following detailed description provided along with illustrations to facilitate the disclosure of the present invention without limiting the same.

### BRIEF DESCRIPTION OF THE DRAWINGS

Fig. 1 is a schematic diagram of function blocks illustrating the gastrointestinal tract diagnosis system of the first embodiment of the instant disclosure.
Fig. 2A is an exploded schematic diagram from a perspective view illustrating the endoscope of the first embodiment of the instant disclosure.
Fig. 2B is an exploded schematic diagram from another perspective view illustrating the endoscope of the first embodiment of the instant disclosure.
Fig. 2C is a schematic diagram from a perspective view illustrating the endoscope without enclosure of the body of the first embodiment of the instant disclosure.
Fig. 2D is a schematic diagram from a cross-section view illustrating the endoscope of the first embodiment of the instant disclosure.
Fig. 2E is a schematic diagram from a perspective view illustrating the endoscope of the first embodiment of the instant disclosure.
Fig. 3A is a schematic diagram from a top view illustrating the magnetic control device of the first embodiment of the instant disclosure.
Fig. 3B is a schematic diagram form a perspective view illustrating the magnetic control device of the first embodiment of the instant disclosure.
Fig. 4A is a schematic diagram illustrating a state of being used for viewing a cavity body by the instant disclosure of the first embodiment.
Fig. 4B is another schematic diagram illustrating a state of being used for viewing a cavity body by the instant disclosure of the first embodiment.
Fig. 4C is a schematic diagram illustrating a usage state of the instant disclosure being used for viewing a stomach.
Fig. 5 is a schematic diagram of a process illustrating the control method of the gastrointestinal tract diagnosis system of the first embodiment of the instant disclosure.
Fig. 6 is a schematic diagram of a process illustrating a control method of the instant disclosure.
Fig. 7 is a schematic diagram of function blocks illustrating the instant disclosure.

### DETAILED DESCRIPTION OF THE PREFERRED EMBODIMENTS

### [First embodiment]

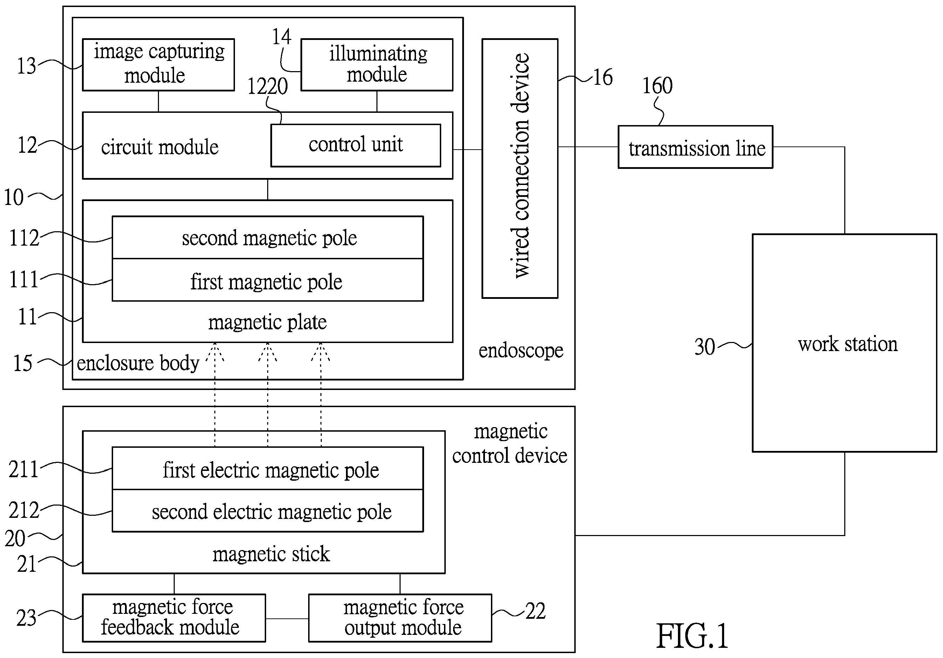

Please refer to Fig. 1, the instant disclosure provides a gastrointestinal tract diagnosis system, at least including: an endoscope 10 and a magnetic control device 20. The endoscope 10 and the magnetic control device 20 can be connected to a work station 30.

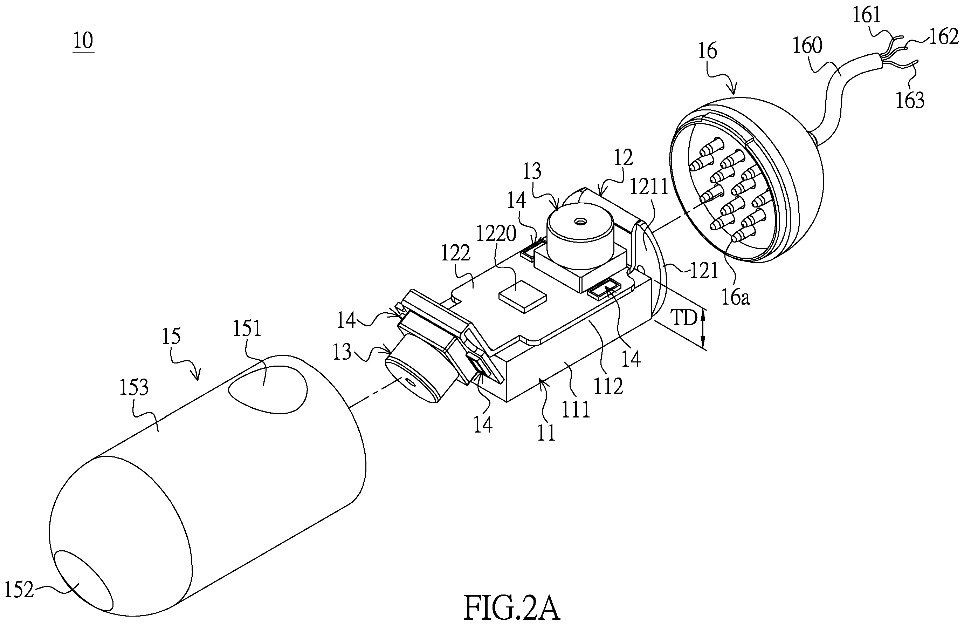

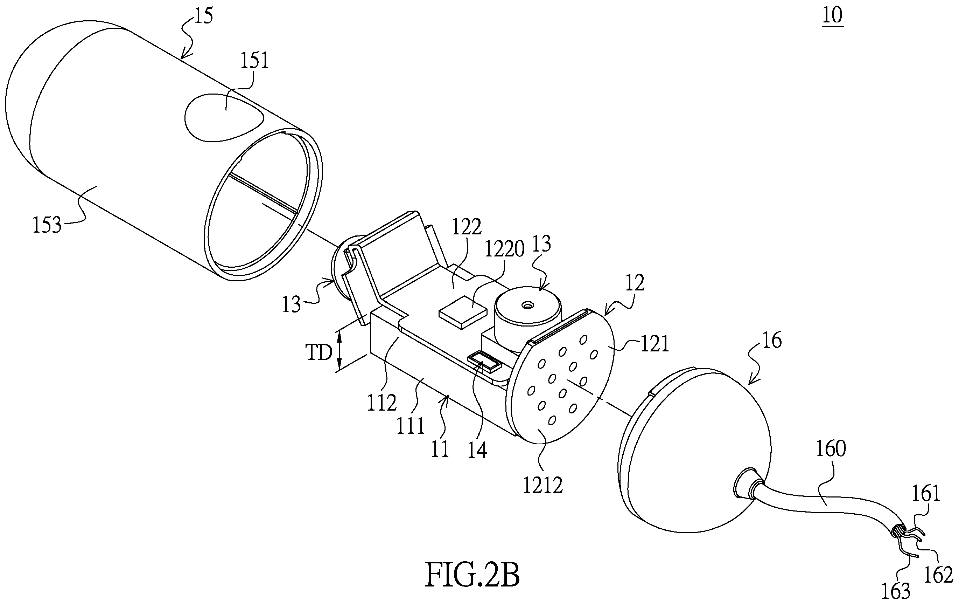

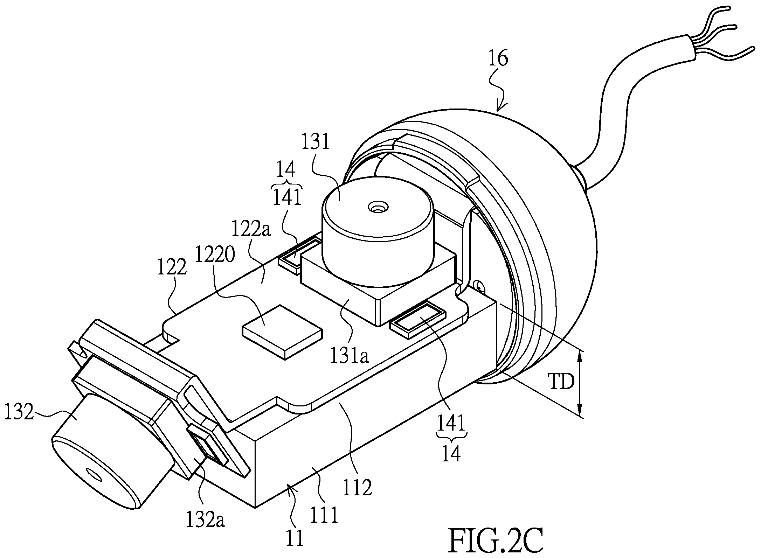

Please refer to Fig. 1, Fig. 2A, Fig. 2B and Fig. 2C, the endoscope 10 has a magnetic plate 11 in flat-plate form therein. A first magnetic pole 111 and a second magnetic pole 112 of the magnetic plate 11 respectively distribute at a lower half portion (label not shown) and an upper half portion (label not shown) of the magnetic plate 11 located along a thickness direction TD of the magnetic plate 11. The magnetic plate 11 can be in the form of a rectangular body, and can be a permanent magnet. Magnetic polarities of the first magnetic pole 111 and the second magnetic pole 112 are opposite to each other. As long as the polarity of the second polarity 112 is opposite to the first polarity 111, the first magnetic pole 111 is not limited to be N polar only or S polar only, and the second magnetic pole 112 is also not limited.

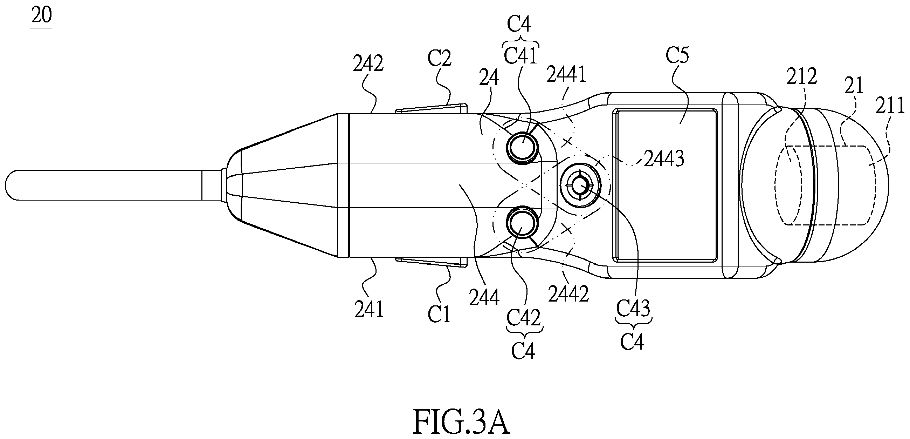

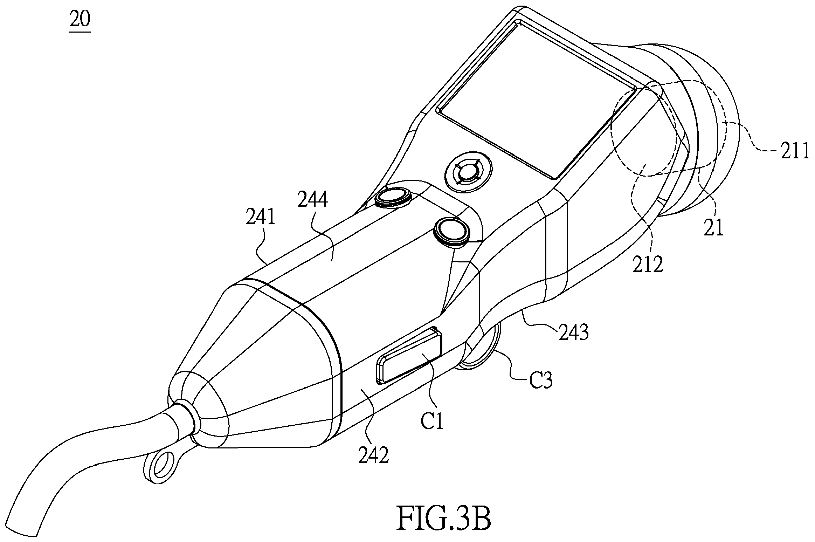

Please refer to Fig. 1, Fig. 3A and Fig. 3B, the magnetic control device 20 includes a magnetic stick 21. At least one electromagnet (not shown) made by an electromagnetic coil (not shown) can be disposed within the magnetic stick 21 so that a first electric magnetic pole 211 is formed at a terminal portion (label not shown) of the magnetic stick 22, and a second electric magnetic pole 212 is formed at a base portion (label not shown) of the magnetic stick 21. In other words, the first electric magnetic pole 211 and the second electric magnetic pole 212 that are formed can be respectively located at two terminals of the electromagnetic coil. The first electric magnetic pole 211 is not limited to be N polar only or to be S polar only. As long as the polarity of the first electric magnetic pole 211 is opposite to that of the second electric magnetic pole 212, and the polarity of the second electric polarity 212 is not limited to be N polar only or to be S polar only, either. The first electric magnetic pole 211 can be used to selectively correspond to, as well as to attract (or to draw) or to repel, one of the first magnetic pole 111 and second magnetic pole 112 of the magnetic plate 11.

Please refer to Fig. 1 illustrating the schematic diagram of function blocks of the instant disclosure. For example, as the first magnetic pole 111 of the magnetic plate 11 is set to be S polar, the first electric magnetic pole 211 can be N polar. The magnetic line of force, also equivalent to names of magnetic field and magnetic force, as schematically represented in the form of the dotted line in Fig. 1, is oriented from a direction from the first electric magnetic pole 211 to the first magnetic pole 111 so that the magnetic stick 21 is able to control the movement and rotation of the endoscope 10 by the attraction of the magnetic force. In order to make the first electric magnetic pole 211 and the second electric magnetic pole 212 of the magnetic stick 21 generate magnetic force, the magnetic control device 20 further includes a magnetic force output module 22 for outputting a predetermined current to supply to the magnetic stick 21. The predetermined current supplied is able to be output and form a predetermined magnetic force on the first electric magnetic pole 211 and the second electric magnetic pole 212 according to the principle of electromagnets. In other words, the magnetic force output module 22 has the magnetic stick 21 generate the predetermined magnetic force for acting on the first magnetic pole 111 and/or the second magnetic pole 112 of the magnetic plate 11 by the way of outputting the predetermined current to the magnetic stick 21. The predetermined magnetic force is applied as a force at a distance to attract or repel the magnetic plate 11. Taking this embodiment for instance, the first electric magnetic pole 211 becomes N polar from which the predetermined magnetic force is generated to be further applied for controlling the movement of the endoscope 10 by attracting the first magnetic pole 111, and exhibiting polarity S, of the magnetic plate 11.

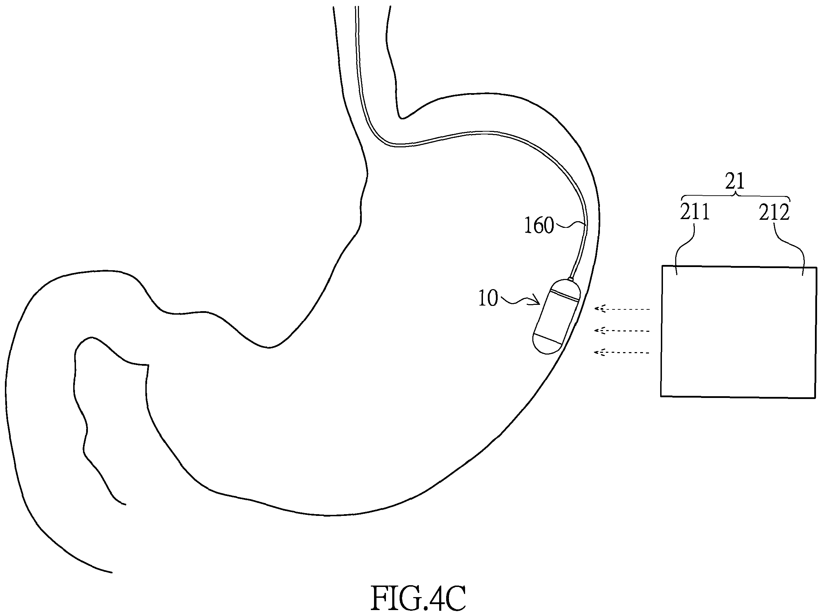

Preferably, the magnetic control device 20 further includes a magnetic force feedback module 23. The magnetic plate 11 can also cause a feedback of magnetic force to a sensor (not shown) within the magnetic stick 21 and change the predetermined current into an offset current, resulting in a variation of the predetermined magnetic force. Subsequently, the magnetic force, for attracting the magnetic plate 11 within the endoscope 10, given off from the magnetic control device 20 can become unstable, leading the endoscope 10 to be in an unstably-swaying condition. Moreover, as the magnetic control device 20 is in the process of drawing the endoscope 10, the magnetic control device 20 and the endoscope 10 get closer and closer to each other, resulting in stronger and stronger attraction force leading to an even closer distance between each other, making it easy for the endoscope 10 to abut the inner wall of the gastrointestinal tract such as the gastric wall (Fig. 4C, label not shown) with over-strength. However, with the aid of the magnetic force feedback module 23, the return of the predetermined default current from the offset current so as to stabilize the output of the predetermined magnetic force can be achieved. Hence, the magnetic force feedback module 23 is helpful for stabilizing the control of the endoscope 10 by the magnetic control device 20. The sway of the endoscope 10 can be decreased. The situation that the gastric wall is hit by over-strength due to excessive magnetic attraction force can also be improved, helping decrease the possible discomfort or other potential physical injury that may be brought on to the check recipients during the process of checking.

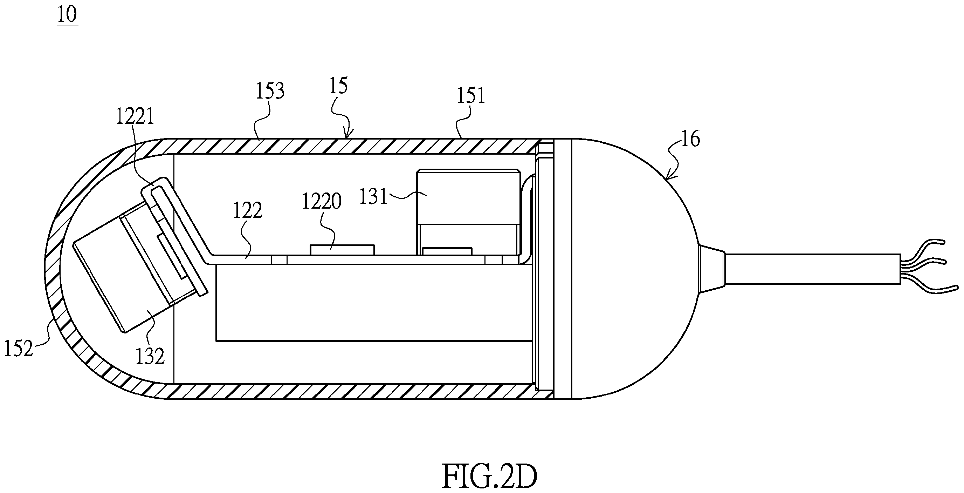

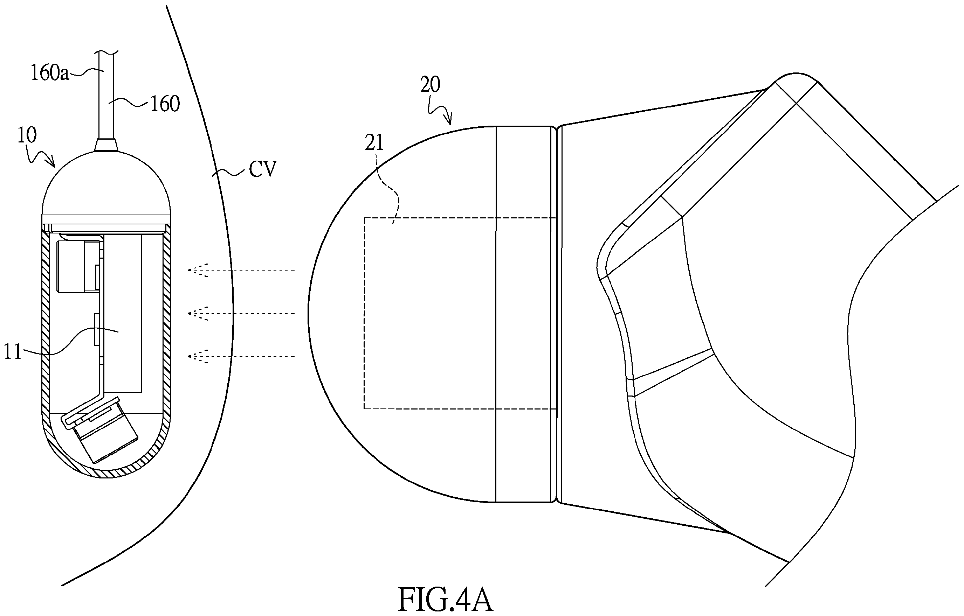

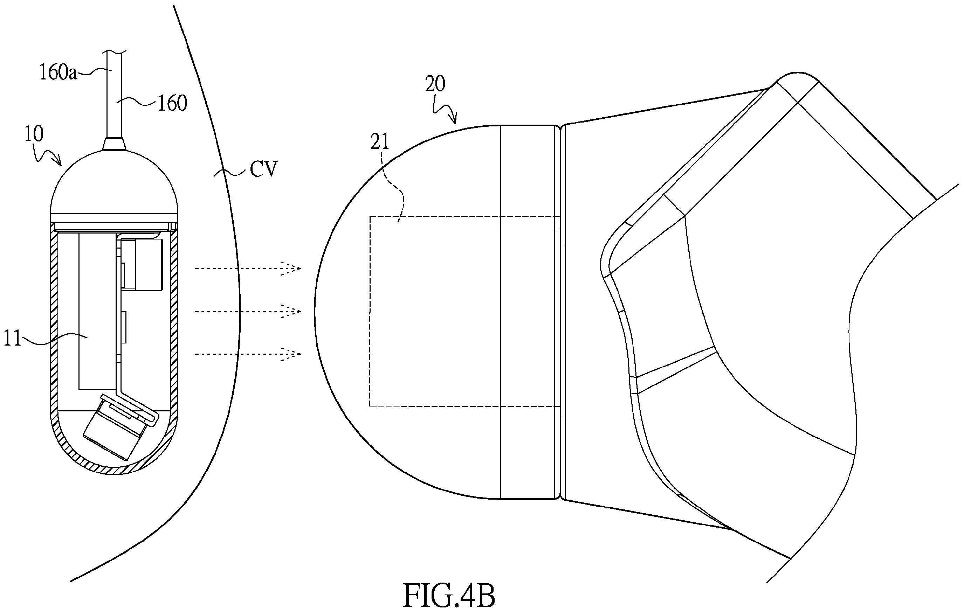

Referring to Fig. 1, 2A and 2B, the endoscope 10 also includes a circuit module 12, an image capturing module 13, an illuminating module 14, an enclosure body 15 and a wired connection device 16. The circuit module 12 at least includes a conductive base 121, a substrate 122 extending from the conductive base 121 and a control unit 1220 being disposed on the substrate 122. The conductive base 121 is electrically assembled to a conductive interface 16a of the wired connection device 16. Preferably, the circuit module 12 can be made of a flexible circuit board. In other words, the substrate 122 can be in the form of a flexible circuit board. Referring to Fig. 2C and Fig. 2D, the image capturing module 13 at least includes a first lens 131 and an image sensor 131a disposed on the substrate 122. The image sensor 131a can be a CCD (charge-couple device) or a CMOS (complementary metal-oxide-semiconductor) for image sensing. The illuminating module 14 includes a first light source 141. The first light source 141 can be disposed on a location adjacent to the first lens 131, providing enough light when the first lens 131 is photo shooting. Preferably, the first lens 131 and the image sensor 131a could be disposed on a surface 122a of the substrate 122 to make it a substantial lateral and horizontal view so that image checking and recording of a lateral 360 degree panorama is able to be done as the endoscope 10 is hung and spins based on the transmission line 160 (referring to the magnetic control device 20 illustrated in Fig. 4A and 4B).

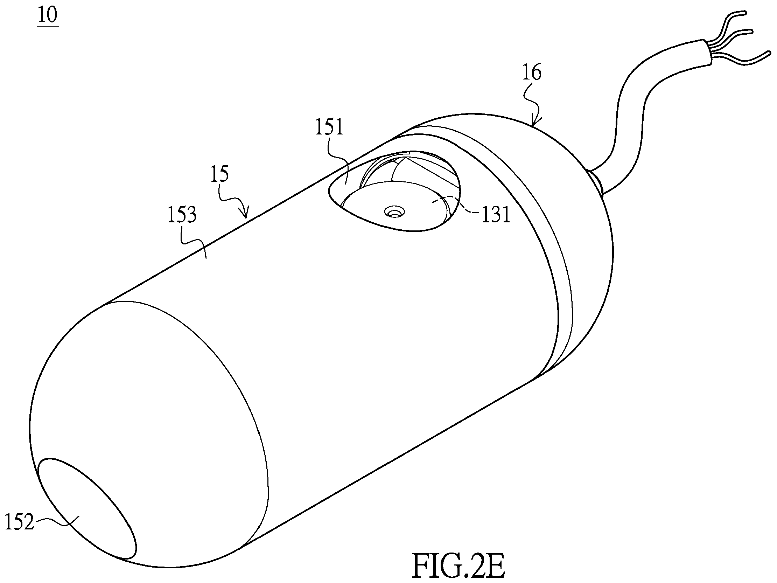

Referring to Fig. 1, Fig.2A, Fig. 2B and Fig. 2E, the enclosure body 15 is assembled with the wired connection device 16 along a direction starting from a first side 1211 of the conductive base 121 toward the wired connection device 16 so that the enclosure body 15 can enclose the circuit module 12 as well as the image capturing module 13, the illuminating module 14 and other related elements or modules having been disposed on the circuit module 12. As the enclosure body 15 is assembled with the wired connection device 16, a water proofing means can be accomplished and provided, rendering the instant disclosure to be a kind of capsule-type endoscope to protect the elements relating to the endoscope 10 within the enclosure body 15 from being destroyed by penetrated liquid. In addition, the wired connection device 16 is assembled with the enclosure body 15 along a direction defined from a second side 1212 of the conductive base 121 to have the ability of being waterproof. The wired connection device 16 electrically connects to the control unit 1220 on the circuit board 12 through the conductive base 121 and the electrical connection is further extended to other elements or modules from the control unit 1220. Preferably, the effect of being waterproof can be achieved by pouring some waterproof gel within any joint (not shown) that may be formed between the wired connection device 16 and the enclosure body 15, but is not limited thereto. A transmission line 160 is extended from the wired connection device 16. The transmission line 160 at least includes a signal transmission line 161 and a power transmission line 162 responsible for transmission of an audio or video signal and providing power, respectively. The endoscope 10 can also equipped with a microphone (not shown) therein to connect with the signal transmission line for audio. Preferably, the transmission line 160 can also include a grounding line 163. Each of the signal transmission line 161, power transmission line 162, and grounding line 163 has a terminal for electrically connecting to the circuit module 12 through the conductive base 121 by the conductive interface 16a. Each of the signal transmission line 161, power transmission line 162 and grounding line 163 has the other terminal being extended therefrom to electrically connect to a work station 30 (shown on Fig. 1) outside of the endoscope 10. The work station 30 can be a PC equipped with a monitor and a receiver (not shown), or the receiver can be a transceiver for receiving signals transmitted from the transmission line 160 and the monitor is for displaying the received signal of the video and the result. The monitor shows the real-time or non-real-time images the endoscope 10 captured. The work station 30 also records and saves the images received from the receiver/transceiver, being able to function as document management. The magnetic plate 11 can be fixed onto the circuit module 12, especially onto a side of the circuit module 12 and parallel to the circuit module 12. Specifically, one magnetic pole of the magnetic plate 11 is adhered to the lower surface of the substrate 122, but is not limited thereto.

Please refer to Fig. 2C. Preferably, the image capturing module 13 can also include a second lens 132 to form a duo lenses module. The second lens 132 is also equipped with an image sensor 132a. As Fig. 2D illustrates, a holder 1221 is slantingly extended from a free end (label not shown), distal to the wired connection device 16, of the substrate 122. The second lens 132 is disposed on the holder 1221, allowing the direction of the second lens 132 to form an angle with a lengthwise axis of the endoscope 10. When the endoscope 10 is controlled by the magnetic control device 20 (as shown in Fig. 4A and 4B) to axially rotate based on the lengthwise axis, the second lens 132 is disposed on the slantingly oriented holder 1221 so that a bigger view can be broadened by the second lens 132. To provide enough light to the second lens 132, a second light source (label not shown) can also be disposed on the holder 1221. Preferably, a light-emitting diode (LED) can be used for the second light source and the first light source 141.

Please refer to Fig. 2D and Fig. 2E. Preferably, the enclosure body 15 includes a first polished region 151, a second polished region 152 and a matte uniformity region 153. The first polished region 151 corresponds to the first lens 131. The first polished region 151 is formed by a polishing on a part of the surface of the enclosure body 15 so that the partial surface of the enclosure body 15 becomes smooth, allowing light to pass through the enclosure body 15 without any blocking to enter the first lens 131 and allowing the image to be captured by the lens clearly. The second polished region 152 is corresponding to the second lens 132. Excepting for the first polished region 151 and the second polished capturing region 152, the rest of the region of the enclosure body 15 can be atomized, for example, but not limited thereto, to treat the surface of the enclosure body 15 by sandblasting so that a matte uniformity region 153 is formed. In such a way, any improper light reflection from the rest of the parts of the surface of the enclosure body 15 except for the first polished region 151 and the second polished region 152 that cause interference to image capturing can be avoided.

Please refer to Fig. 1, 3A and 3B, the magnetic device 20 further includes a holding portion 24 connected with the base portion of the magnetic stick 21. The holding portion 24 includes a right side 241, a left side 242, a belly portion 243 and a back portion 244. The right side 241 has a first booting button C1 used for being pressed. The left side 242 has a second booting button C2 used for being pressed. The belly portion 243 has a ring C3 thereon. The ring C3 is preset in an off-stated default. The back portion 244 has manipulation buttons C4 and a screen C5 thereon. The manipulation buttons C4 are pre-configured in an off-stated default. Both the first booting button C1 and the second booting button C2 can be prepressed to unlock the off-stated default of the ring C3 and the manipulation buttons C4. In detail, users have to prepress either the first booting button C1 or the second booting button C2 so as to active the control function of the ring C3 and the manipulation buttons C4. The belly portion 243 and the magnetic stick 21 form an angle between 155 degree and 175 degree, making it fairly similar to the holding portion 24 and the magnetic stick 21 that form an angle between 155 degree and 175 degree to form a gun-like structure. It is convenient for users to hold and control direction of the magnetic control device 20. Take the gun-like structure for example, when right-handed users hold the holding portion 24, the first booting button C1 will be firstly and simultaneously pushed by the palm of the right hand, but the second booting button C2 will not be pressed. Hence, the off-stated default of the ring C3, manipulation buttons C4 and screen C5 would be unlocked to recover the control function. The forefinger of the right hand can easily hook to the ring C3 and control the rotation of the first electric magnetic pole 211 and the second electric magnetic pole 212 of the magnetic stick C3 by triggering the ring C3 forward or backward so as to drive the endoscope 10 to rotate or move. For instance, the magnetic stick 21 can be driven to rotate clockwise by pushing the ring C3 forward, or can be driven to rotate counter clockwise by pulling the ring C3 backward, helping the endoscope 10 to be positioned in a specific direction to obtain the desired sight for shooting or observing. As the Fig. 3A shows, the thumb of the right hand could access to the manipulation buttons C4 to press the left key C41 of the manipulation buttons C4 and press the menu key C43 of the manipulation buttons C4 without resulting in unstable holding. In other words, the left key C41 can be arranged on the first location 2441 of the back portion 244, and the first location 2441 can be pressed by the thumb of the right hand. Likewise, the second booting button C2 on the left side 242 and the right key C42 on the manipulation buttons C4 is suitable for left-handed users. The manipulation circumstance for left-handed users is analog to that for the right-handed users, except the different orientations of use resulting from right hand and the left hand and unnecessary details for left handed users will not be introduced here. The right key C42 can be arranged on a second location 2442 on the back portion 244. The second location 2442 can be pressed by the thumb of the left hand. Preferably, the menu key C43 can be arranged on a third location 2443 on the back portion 244. The third location 2442 is a location that can be pressed by the thumbs of the right hand and the left hand. The third location would be a place defined by the overlap of the first location 2441 and the second location 2442. Referring to Fig. 1 and 3A, the left key C41 and the right key C42 are mainly used for controlling and initiating the shooting of the image capturing module 13, therefore the left key C41 and the right key C42 can further be a first two-stage button and a second two-stage button, respectively. They are similar to shutter keys of a camera, when being clicked down to the first stage, focusing of the endoscope 10 is carried out, and when being clicked down to the second stage, image shooting of the endoscope 10 is performed. For the convenience of saving images, a memory card reader (not shown) can be further arranged on the holding portion 24 to access the image file anytime however work station 30 can be responsible for saving and recording work.

Please refer to Fig. 1, 4A, 4B and 5, according to the above technical contents, the instant disclosure further provides a control method of a gastrointestinal tract diagnosis system, at least including the following steps.

Step S101: providing an endoscope 10 having a transmission line 160 and a magnetic plate 11, and delivering the endoscope 10 into a cavity body CV with a first section 160a of the transmission line 160 and a second section (not shown) of the transmission line 160 being left outside of the cavity body CV and being connected to a work station 30. The cavity body CV could be a cavity of a living body or a bag-shaped cavity with two openings respectively being opened at two terminals on the cavity and connecting to the cavity, for example, the stomach, but not limited thereto, therefore the cavity could be one of a non-living body. Taking the stomach for demonstration, preferably, the endoscope 10 with the first section 160a of the transmission line 160 can be delivered into the stomach by swallowing. Thus, the transmission line 160 would be a soft line made of insulation material having high biocompatibility. The transmission line 160 is different to the hard tube of the known endoscope and it would be better for the transmission line 160 to have a smaller diameter to avoid discomfort caused on check recipients. Preferably, the transmission line 160 could have a diameter ranging from 1.4 to 2.4 centimeter (cm), and this diameter range basically approaches to the diameter of a common adult. Nevertheless, the above diameters are merely for being references and are not limited thereto. In particular, as long as the diameter of the transmission line 160 conforms to that of a person's esophagus, the esophagus feeling of being invaded by foreign material caused to a swallower is largely decreased, but the button limit of the diameter is not limited and diameter of the transmission line 160 would be viewed at its best size. It is believed that feeling of fear and being invaded could be decreased by swallowing the endoscope 10 with a transmission line 160 at the best diameter. Besides, it also helps to allow a section of the transmission line 160 to get in the cavity body CV along with the endoscope 10 and the other section (not shown) of the transmission 160 would be left outside of the cavity body CV. The retaking of the instant disclosure after being used would not be a problem.

Step S103 includes: providing a magnetic control device 20 at least having a magnetic stick 21 and a magnetic force output module 22, and activating the magnetic control device 20 to allow the magnetic force output module 22 to generate a predetermined magnetic force so that one of the two electric magnetic poles could be used to electro-magnetically attract the magnetic plate 11 within the endoscope 10. Then the observation of the cavity body CV could be done by attracting / drawing the endoscope 10 toward an appropriate view by the magnetic control device 20.

Step S105 includes: adjusting the movement, rotation and view field of the endoscope 10 inside the cavity body CV by means of the magnetic control device 20 to record images of the inner wall of the cavity body CV.

Step S107 includes: repeating step (b) to step (c) for a number of times till the endoscope 20 finishes recording images of the inner wall of the cavity body CV. It is worth noting that during the process S107, the endoscope 10 carries out the recording basically after being drawn to an appropriate location inside the cavity body CV corresponding to the nidus and unnecessary redundant shooting as well as image recording would not result. The trouble of screening the huge amount of image data to know if any images have been recorded of any desired image of a nidus would be avoid.

Step S109 includes: terminating the magnetic control device 20 to cease the predetermined magnetic force.

Step S111 includes: pulling the second section (not shown) of the transmission line 160 to render the endoscope 10 moving from inside of the cavity body CV to outside of the cavity body CV, so as to retake the endoscope 10. Because the instant disclosure has the transmission line 160, the problem of it being difficult to get the endoscope 10 out of the cavity body CV can be overcome.

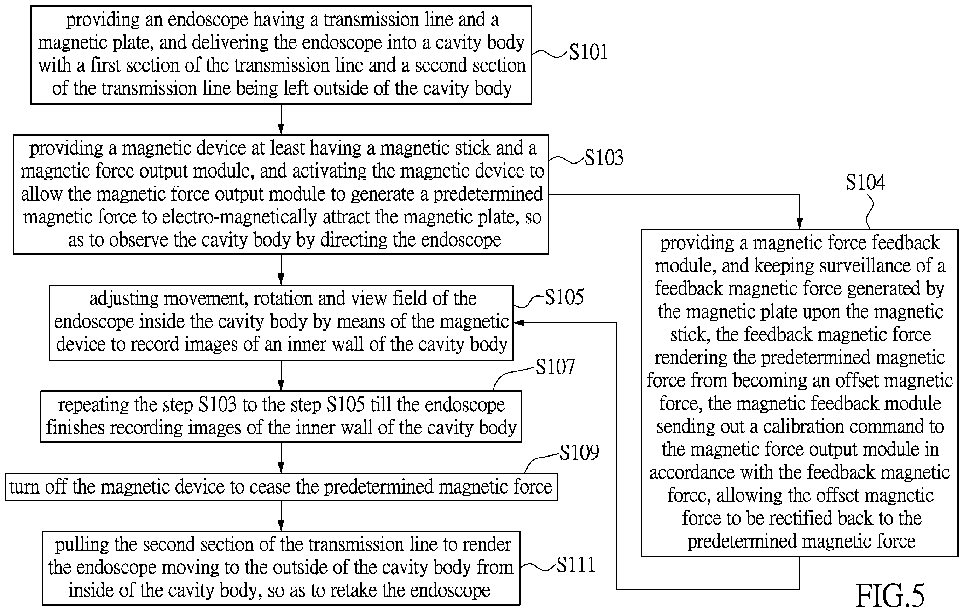

Preferably, as shown in Fig. 1 and 5, when the step S103 is progressed to the step S105, the following step S104 is further included: providing a magnetic force feedback module 23, and keeping surveillance of a feedback magnetic force generated by the magnetic plate 11 upon the magnetic stick 21, the feedback magnetic force rendering the predetermined magnetic force from becoming an offset magnetic force, the magnetic feedback module 23 sends out a calibration command to the magnetic force output module 22 in accordance with the feedback magnetic force, allowing the offset magnetic force to be rectified back to the predetermined magnetic force. As the step S103 to the step S105 is repeated within the above step S107, the step S104 can also be added between step S103 and S105 for being repeated. In addition, during progression of step S103 to step S105, real-time image recording and displaying according to the view of the endoscope 10 can be done by means of the work station 30 being connected with the transmission line 160. It is understood that the instant disclosure has the advantages of shooting and displaying real-time images.

### [second embodiment]

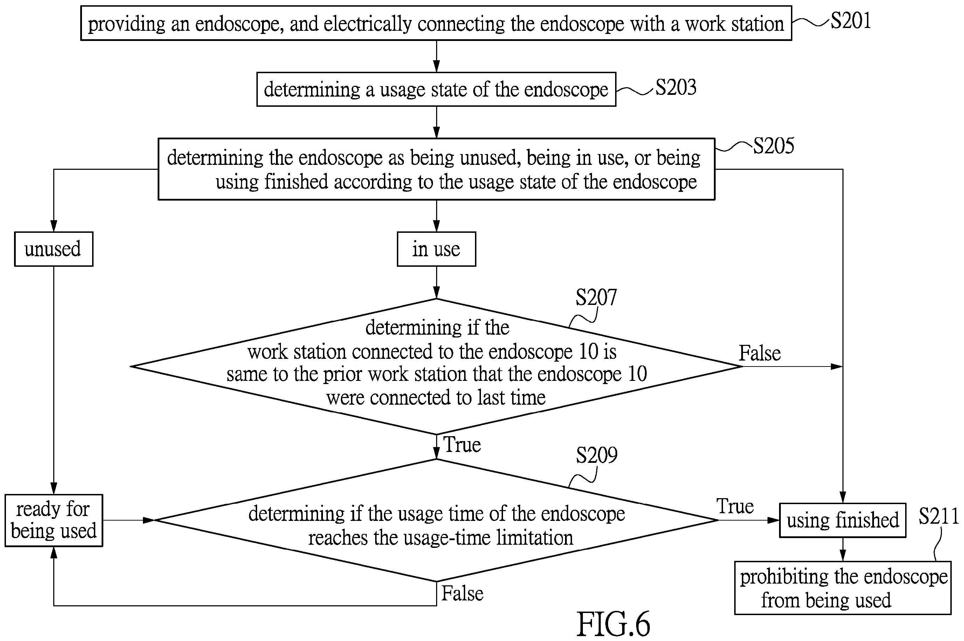

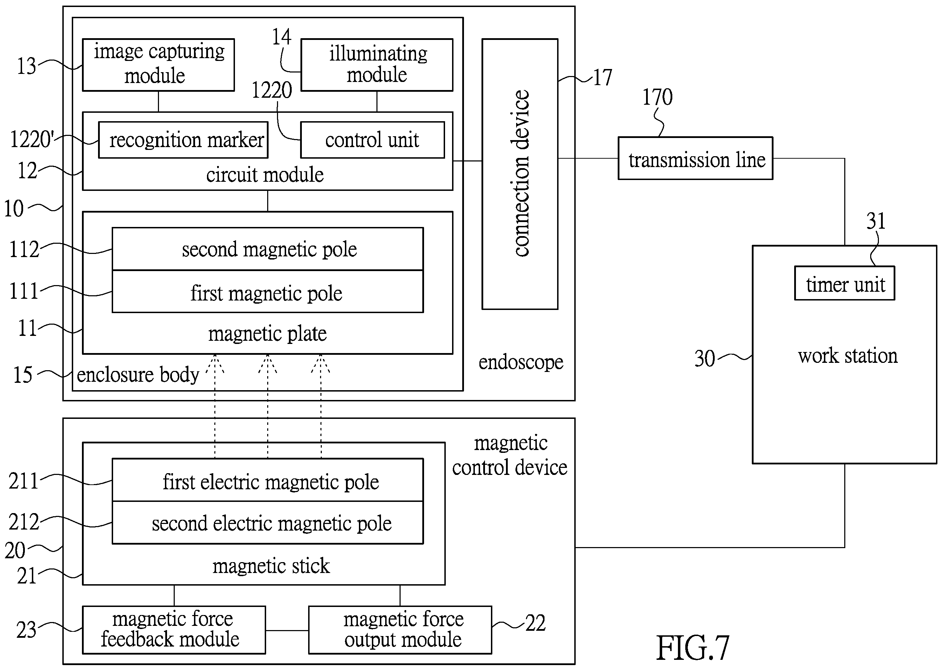

Referring to Figs. 6 and 7, the instant disclosure further provides a control method for a gastrointestinal tract diagnosis system with disposable endoscope. The control method at least includes the following steps: step S201: providing an endoscope 10, and connecting the endoscope 10 with a work station 30. Step S203: determining a usage state of the endoscope 10. Step S205: determining the endoscope 10 as being not yet used, being in use, or being out of use according to the usage state of the endoscope 10. If the usage state of the endoscope 10 is determined as being out of use, the step S211 is directly carried out to prohibit the usage of the endoscope.

If the usage state of the endoscope 10 is determined as being not yet used, go on to carry out the step S209 to determine if a usage time of the endoscope 10 reaches a usage-time limitation till the usage time reaches the usage-time limitation, then determining the usage state of the endoscope 10 as being out of use, subsequently carrying out the step S211 to prohibit the usage of the endoscope 10.

If the usage state of the endoscope 10 is determined as being in use, go on to determine if the work station 30 that the endoscope is connected to is the same as a prior work station (not shown) that the endoscope 10 was connected to last time. If the work station 30 is different from the prior work station having been connected to last time, determine the endoscope 10 as being out of use and carry out the step S211 to prohibit the endoscope 10 from being used. On the other hand, if the work station 30 is confirmed to be the same as the prior work station 30 that the endoscope 10 was connected to last time by carrying out stepS207, go on to carry out the step S209 to determine if the usage time of the endoscope 10 reaches the usage-time limitation. If the result of the step S209 is "false", the endoscope 10 can be continued to be operated and the step S209 is returned to make determination of the endoscope 10 till the usage time of the endoscope 10 reaches the usage-time limitation which means the result of the step S209 is "true" so that the usage state of the endoscope 10 is determined as being out of use and the step S211 is carried out to prohibit the endoscope 10 from being used.

Preferably, the step S207, to determine if the work station 30 being connected to by the endoscope 10 is the same as the prior work station that the endoscope 10 was connected to last time, is based on the checking of the machine identity (e.g. serial number of firmware) of the work station 30 and the machine identity of the prior work station. In detail, the prior work station 30 being connected to can be matched based on a recognition marker 1220' of the endoscope 10, and the recognition marker 1220' can be added with the machine identity of the prior work station by means of the prior work station. Thus, the work station 30 can determine if the machine identity of the prior work station is the same as that of the work station 30 present to determine if the work station 30 the endoscope 10 being connected to the same as the prior work station. The above disclosure is a preferable demonstration but is not limited thereto.

Preferably, the endoscope 10 can be preset to have a period of usage-time limitation. In other words, the usage of the endoscope 10 can be restricted by the usage-time limitation. The usage-time limitation can be delimited to at least include an initial value and a final value. The aforementioned step S201 further include steps of initiating a timer unit 31 of the work station 30 to generate a timing value according to the timer unit 31. The timer unit 31 can run the work of timing in a way of counting up or counting down. Despite counting up or counting down, an initial value and a final value can be generated. Thus, the way that the timer unit 31 works in the instant embodiment is not limited to ways of counting up or counting down. If the timing value is equal to the initial value, the endoscope 10 is determined as being unused. If the timing value is equal to or beyond the final value, the usage state of the endoscope 10 is determined as being using finished in step S205. If the timing value is between the initial value and the final value, the usage state of the endoscope 10 is determined as being in use. Preferably, the aforementioned usage-time limitation can be preset depending to demands, e. g. usage-time limitation of 10 to 30 minutes, as the products are being finalized and leaving the factory but is not limited thereto. However, if it is to be used for viewing the gastrointestinal tract, generally speaking, 10 minutes of usage-time limitation would be enough. Furthermore, one of the ways to prohibit the endoscope 10 from being used by means of the work station 30 as well as software installed therein is to have the endoscope 10 disabled, to have the endoscope 10 unable to be activated, or to have the fuse (not shown) in the endoscope 10 cut by inputting an instantaneous electrical power of low voltage and high current, so as to prohibit the endoscope 10 from being used. The aforementioned fuse can be installed in the circuit of the circuit module 12 but is not limited thereto. The elements inside the endoscope 10 of this embodiment, e.g. the circuit module, can also be waterproofingly enclosed by the enclosure body 15 as introduced in the first embodiment. Because the aforementioned control method is realized through the satisfying of some requirements to forcibly prohibit the endoscope 10 from being used, the endoscope 10 is also forcibly turned into an endoscope 10 that must be discarded and replaced, so as to meet the purpose of having a disposable endoscope, of the instant disclosure.

Please refer to Fig. 7, another variant derived according to the first embodiment and the aforementioned control method. The different technical features of the variant of the second embodiment and the first embodiment can be commonly applied to each other. The instant embodiment provides a gastrointestinal tract diagnosis device with disposable endoscope, at least including an endoscope 10, and a work station 30. The work station 30 is connected to the endoscope 10. The endoscope 10 has a circuit module 12, an image capturing module 13, an illuminating module 14 and an enclosure body 15.

What is different from the first embodiment is that the circuit module 12 of the second embodiment at least includes a substrate 122, and a control unit 1220 as well as a recognition marker 1220' that are disposed in the substrate 122. The work station 30 can be used for connecting the endoscope 10 to read/access or write/add the machine identity of the work station 30 from or in the recognition marker 1220', so as to match with the endoscope 10. The recognition marker 1220' can be firmware for the purpose of recognition that is burned on the substrate 122 to hold information for recognition, such as a serial number or symbol, related to identifying the endoscope 10. In other words, the recognition marker 1220' not only can carry recognition information related to the endoscope 10 but also can be added or written in with information about the machine identity of the work station 30, allowing the work station 30 to determine if the endoscope 10 has connected to a prior work station different to the present work station 30 according to the recognition marker 1220' and the machine identity inside the recognition marker 1220'.

Referring to Fig. 2C, the similar part of the instant embodiment and the first embodiment is that the image capturing module 13 at least includes a first lens 131 as well as an image sensor 131a that are disposed on the substrate 122. The illuminating module 14 at least includes a first light source 141 disposed on the substrate 122. The enclosure body 15 is the same to the one shown in Fig. 5A except that the enclosure body 15 of the instant embodiment is not deemed to have to connect with a wired connection device. The enclosure body 15 connects to the connection device 17 of which the function is for waterproofingly enclosing the circuit module 122, image capturing module 13 and the illuminating module 14. In other words, the connection device 17 is not limited to have to be equipped with a transmission line 160 as the wired connection device 16 shown in Fig. 2A is. The connection device 17 can only be assembled with the enclosure body 15, rendering the endoscope 10 to become a wireless endoscope capsule. Therefore, the endoscope 10 is able to undergo wireless connection with the work station 30 under the wireless condition, however, is not limited thereto. Hence, the connection device 17 can also connect to the work station 30 through a wired manner.

Preferably, the work station 30 also has a timer unit 31. The work station 30 is able to determine if the usage state of the endoscope 10, carrying the same recognition marker 1220' as the work station 30, reaches or is over the preset usage-time limitation. Once the usage state of the endoscope 10 reaches or is over the time limit, the endoscope 10 is disabled in a way such as the aforementioned control method. The work station 30 can also determine if the endoscope 10 had been connected to a prior work station different to the present work station 30 according to the recognition marker 1220' and the information of machine identity written in the recognition marker 1220'. A recognition unit (not shown) in the work station 30 can be used to identify the recognition marker 1220' to achieve the determination but is not limited thereto.

In the instant embodiment, a magnetic plate 11 can also be disposed inside the endoscope 10 and collocate with a magnetic control device 20 and the magnetic stick 21, the magnetic force output module 22, as well as the magnetic force feedback module 23 to work in a way similar to the aforementioned embodiments disclosed. In other words, the device as being disclosed in the first embodiment can also include some elements of the device as being demonstrated in the instant embodiment, making the endoscope 10 function as a disposable element. However, the instant embodiment still can escape from the typical model of the first embodiment to become a gastrointestinal tract diagnosis system with disposable endoscope independent from the first embodiment. Please refer to Fig. 4C. Fig. 4C illustrates a usage state of the instant disclosure being used for viewing a stomach. The magnetic stick 21, which has a first electric magnetic pole 211 and a second electric magnetic pole 212, is illustrated in a form of a rectangle. The first electric magnetic pole 211 is closer to the stomach (label not shown) than the second electric magnetic pole 212 is in Fig. 4C. The first electric magnetic pole 211 attracts / draws the endoscope 10 inside the stomach in a way of distal force and the movement of the endoscope 10 in the stomach is under control.

To sum up all of the above embodiments, more stable control of the endoscope can be achieved. The inappropriate physical harm to the cavity being viewed resulting from the endoscope can be avoided. In addition the instant disclosure is easy to be taken back or recycled. Therefore as the instant disclosure is delivered into the living body, the problem of being difficult to take the endoscope out will not be caused. Thus, unnecessary surgical as well as medical source waste will not be caused. After being recovered, the function of the endoscope can be forcibly disabled to solve the problem that endoscopes being repetitively used in checking gastrointestinal tracts of non-single-specific cases and the problem that the subsequent sterilizing cannot be guaranteed. The descriptions illustrated supra set forth simply the preferred embodiments of the present invention; however, the characteristics of the present invention are by no means restricted thereto. All changes, alterations, or modifications conveniently considered by those skilled in the art are deemed to be encompassed within the scope of the present invention delineated by the following claims.

## Claims

1. A gastrointestinal tract diagnosis system, at least comprising:
an endoscope (10) having a magnetic plate (11) therein, the magnetic plate (11) having a first magnetic pole (111) and a second magnetic pole (112) respectively distributed at a lower half portion and an upper half portion of the magnetic plate (11) located along a thickness direction (TD) of the magnetic plate (11); and
a magnetic control device (20) including:
a magnetic stick (21) including a first electric magnetic pole (211) at a terminal portion of the magnetic stick (21) and a second electric magnetic pole (212) located at a base portion of the magnetic stick (21);
a magnetic force output module (22) for being used to output a predetermined current to the magnetic stick (21) so as to allow the magnetic stick (21) to generate a predetermined current generating a predetermined magnetic force for interacting with the first magnetic pole (111) and the second magnetic pole (112); **characterized in,**
**that**
the predetermined magnetic force is adapted to draw the magnetic plate (11) to control the movement of the endoscope (10),
and the magnetic plate (11) is adapted to cause a feedback magnetic force to a sensor of the magnetic stick (21), converting the predetermined current into an offset current, and
**that** the magnetic control device (20) further includes
a magnetic force feedback module (23), wherein
the magnetic force feedback module (23) is adapted to calibrate the offset current, resuming the offset current back to the predetermined current, so as to stabilize the predetermined magnetic force.

2. The gastrointestinal tract diagnosis system according to claim 1, wherein the endoscope (10) includes:
a circuit module (12) at least including a conductive base (121), a substrate (122) extending from the conductive base (121) and a control unit (1220) disposed on the substrate (122);
an image capturing module (13) at least including a first lens (131) and an image sensor (131a) disposed on the substrate (122);
an illuminating module (14) at least including a first light source (141) disposed on the substrate (122);
an enclosure body (15); and
a wired connection device (16) having a conductive interface (16a) and a transmission line (160), one terminal of the transmission line (160) connecting with the conductive interface (16a) and the other terminal of the transmission line (160) connecting with a work station (30),
wherein the conductive interface (16a) electrically connects with the control unit (1220) on one side of the conductive base (121);
the enclosure body (15) is waterproof and is assembled to the wired connection device (16) from the other side of the conductive base (121), enclosing the circuit module (12), the image capturing module (13) and the illuminating module (14); and
the magnetic plate (11) is fixed on the circuit module (12).

3. The gastrointestinal tract diagnosis system according to claim 2, wherein a holder (1221) is slantingly extended from a free end of the substrate (122), and the first lens (131) is disposed on the holder (1221).

4. The gastrointestinal tract diagnosis system according to claim 3, wherein the image capturing module (13) includes a second lens (132), the second lens (132) being disposed on a surface (122a) of the substrate (122).

5. The gastrointestinal tract diagnosis system according to claim 4, wherein the enclosure body (15) includes a first polished region (151), a second polished region (152) and a matte uniformity region (153) thereon, wherein the first polished region (151) is corresponded to the first lens (131) and the second polished region (152) is corresponded to the second lens (132).

6. The gastrointestinal tract diagnosis system according to claim 1, wherein the magnetic control device (20) includes:
a holding portion (24) connected with the base portion of the magnetic stick (21), the holding portion (24) including:
a first side (241) with a first booting button (C1) preset in an off-stated default thereon;
a second side (242) with a second booting button (C2) preset in an off-stated default thereon;
a belly portion (243)with a ring (C3) thereon for controlling a spin state of the magnetic stick (21); and
a back portion (244)with manipulation buttons (C4) and a screen (C5) thereon, wherein the first booting button (C1) and the second booting button (C2) are for being pressed firstly for respectively unlocking the off-stated default of the ring (C3) and the manipulation buttons (C4).

7. The gastrointestinal tract diagnosis system according to claim 6, wherein the manipulation buttons (C4) include buttons of a menu (C43), a first key (C41) and a second key (C42),
wherein the first key (C41) is arranged on a first location (2441) of the back portion (244), so as to be capable of being pressed by a thumb of a right hand;
the second key (C42) is arranged on a second location (2442) of the back portion (244), so as to be capable of being pressed by a thumb of a left hand; and
the buttons of the menu (C43) are arranged on a third location (2443) of the back portion (244), so as to be capable of being pressed by the thumb of either the left hand or the right hand.

8. The gastrointestinal tract diagnosis system according to claim 6, wherein the belly portion (243) and the magnetic stick (21) form an angle between 155 degree and 175 degree.

9. The gastrointestinal tract diagnosis system according to claim 7, wherein the belly portion (243) and the magnetic stick (21) forms an angle between 155 degree and 175 degree.

10. The gastrointestinal tract diagnosis system according to claim 7, wherein the first key (C41) and the second key (C42) are a first key of two-stage pushing and a second key of two-stage pushing, respectively.

11. A control method of a gastrointestinal tract diagnosis system, at least comprising the following steps:
(a) providing an endoscope (10) having a transmission line (160) and a magnetic plate (11), and delivering the endoscope (10) into a cavity body (CV) with a first section (160a) of the transmission line (160) and a second section of the transmission line (160) being left outside of the cavity body (CV);
(b) providing a magnetic device at least having a magnetic stick (21) and a magnetic force output module (22), and activating the magnetic device to allow the magnetic force output module (22) to generate a predetermined magnetic force to electro-magnetically attract the magnetic plate (11), so as to observe the cavity body (CV) by directing the endoscope (10);
(c) providing a magnetic force feedback module (23), and keeping surveillance of a feedback magnetic force generated by the magnetic plate (11) upon the magnetic stick (21), the feedback magnetic force rendering the predetermined magnetic force becoming an offset magnetic force, the magnetic force feedback module (23) sending out a calibration command to the magnetic force output module (22) in accordance with the feedback magnetic force, allowing the offset magnetic force to be rectified back to the predetermined magnetic force
(d) adjusting movement, rotation and view field of the endoscope (10) inside the cavity body (CV) by means of the magnetic device to record images of an inner wall of the cavity body (CV);
(e) repeating the step (b) to the step (c) till the endoscope (10) finishes recording images of the inner wall of the cavity body (CV);
(f) turning off the magnetic device to cease the predetermined magnetic force; and(g) pulling the second section of the transmission line (160) to render the endoscope (10) moving to the outside of the cavity body (CV) from inside of the cavity body (CV), so as to retake the endoscope (10).

## Patentansprüche

1. Gastrointestinaltraktdiagnosesystem, zumindest umfassend:
ein Endoskop (10) mit einer sich darin befindlichen Magnetplatte (11), wobei die Magnetplatte (11) einen ersten Magnetpol (111) und einen zweiten Magnetpol (112) aufweist, die jeweils an einem unteren Halbabschnitt und einem oberen Halbabschnitt der Magnetplatte (11) entlang einer Dickenrichtung (TD) der Magnetplatte (11) angeordnet sind, und
eine magnetische Steuervorrichtung (20) umfassend:
einen Magnetstab (21) mit einem ersten elektrischen Magnetpol (211) an einem Anschlußabschnitt des Magnetstabs (21) und einem zweiten elektrischen Magnetpol (212), der an einem Basisabschnitt des Magnetstabs (21) angeordnet ist,
ein magnetisches Kraftausgabemodul (22), das verwendbar ist, um einen vorbestimmten Strom an den Magnetstab (21) auszugeben, um es dem Magnetstab (21) zu ermöglichen, einen vorbestimmten Strom zu erzeugen, der eine vorbestimmte magnetische Kraft erzeugt, um mit dem ersten Magnetpol (111) und dem zweiten Magnetpol (112) zusammenzuwirken,
**dadurch gekennzeichnet, dass**
die vorbestimmte magnetische Kraft dazu ausgebildet ist, die Magnetplatte (11) zu ziehen, um die Bewegung des Endoskops (10) zu steuern,
und die Magnetplatte (11) dazu ausgebildet ist, eine Rückkopplungsmagnetkraft auf einen Sensor des Magnetstabes (21) bewirkt, den vorbestimmten Strom in einen Offsetstrom umzuwandeln und
dass die magnetische Steuervorrichtung (20) ferner ein Magnetkraftrückkopplungsmodul (23) umfasst, wobei das Magnetkraftrückkopplungsmodul (23) ausgebildet ist, um den Offsetstrom zu kalibrieren und den Offsetstrom wieder auf den vorbestimmten Strom zurückzusetzen, um so die vorbestimmte Magnetkraft zu stabilisieren.

2. Gastrointestinaltraktdiagnosesystem nach Anspruch 1, wobei das Endoskop (10) umfasst:
ein Schaltkreismodul (12), das zumindest eine leitfähige Basis (121), ein sich von der leitfähigen Basis (121) erstreckendes Substrat (122) und eine auf dem Substrat (122) angeordnete Steuereinheit (1220) aufweist, ein Bildaufnahmemodul (13), das zumindest eine erste Linse (131) und einen auf dem Substrat (122) angeordneten Bildsensor (131a) aufweist, ein Beleuchtungsmodul (14), das zumindest eine auf dem Substrat (122) angeordnete erste Lichtquelle (141) aufweist,
einen Gehäusekörper (15), und
eine drahtgebundene Verbindungsvorrichtung (16) mit einer leitfähigen Schnittstelle (16a) und einer Übertragungsleitung (160), wobei ein Anschluss der Übertragungsleitung (160) mit der leitfähigen Schnittstelle (16a) und dem anderen Anschluss der Übertragungsleitung (160) verbunden ist, die mit einer Arbeitsstation (30) verbunden ist,
wobei die leitfähige Schnittstelle (16a) elektrisch mit der Steuereinheit (1220) auf einer Seite der leitfähigen Basis (121) verbunden ist,
der Gehäusekörper (15) wasserdicht ist und an der verdrahteten Verbindungseinrichtung (16) von der anderen Seite des leitenden Bodens (121), die das Schaltkreismodul (12), das Bilderfassungsmodul (13) und das Beleuchtungsmodul (14) umschließt, montiert ist, und
die Magnetplatte (11) auf dem Schaltkreismodul (12) befestigt ist.

3. Gastrointestinaltraktdiagnosesystem nach Anspruch 2, wobei sich ein Halter (1221) schräg von einem freien Ende des Substrats (122) erstreckt und die erste Linse (131) auf dem Halter (1221) angeordnet ist.

4. Gastrointestinaltraktdiagnosesystem nach Anspruch 3, wobei das Bildaufnahmemodul (13) eine zweite Linse (132) aufweist, wobei die zweite Linse (132) auf einer Oberfläche (122a) des Substrats (122) angeordnet ist.

5. Gastrointestinaltraktdiagnosesystem nach Anspruch 4, wobei der Gehäusekörper (15) einen ersten polierten Bereich (151), einen zweiten polierten Bereich (152) und einen darauf befindlichen matten gleichförmigen Bereich (153) aufweist, wobei der erste polierte Bereich (151) der ersten Linse (131) zugeordnet ist und der zweite polierte Bereich (152) der zweiten Linse (132) zugeordnet ist.

6. Gastrointestinaltraktdiagnosesystem nach Anspruch 1, wobei, die magnetische Steuervorrichtung (20)
einen Halteabschnitt (24), der mit dem Basisabschnitt des Magnetstabs (21) verbunden ist, aufweist, wobei der Halteabschnitt (24) aufweist:
eine erste Seite (241) mit einer ersten Boottaste (C1) darauf, die in einen ausgeschalteten Zustand voreingestellt ist,
eine zweite Seite (242) mit einer zweiten Boottaste (C2) darauf, die mit einen ausgeschalteten Zustand voreingestellt ist,
einen Bauchabschnitt (243) mit einem darauf befindlichen Ring (C3) zum Steuern eines Spinzustands des Magnetstabs (21), und
einen Rückenabschnitt (244) mit Manipulationstasten (C4) und einen Bildschirm (C5) darauf, wobei die erste Boottaste (C1) und die zweite Boottaste (C2) zunächst gedrückt werden, um den ausgeschalteten Zustand des Rings (C3) und der Manipulationstasten zu entriegeln (C4).

7. Gastrointestinaltraktdiagnosesystem nach Anspruch 6, wobei die Manipulationstasten (C4) Menütasten (C43), eine erste Taste (C41) und eine zweite Taste (C42) umfassen,
wobei die erste Taste (C41) an einer ersten Stelle (2441) des hinteren Teils (244) angeordnet ist, um von einem Daumen einer rechten Hand gedrückt zu werden,
die zweite Taste (C42) an einer zweiten Stelle (2442) des hinteren Teils (244) angeordnet ist, um durch einen Daumen einer linken Hand gedrückt zu werden, und
die Menütasten (C43) an einer dritten Stelle (2443) des hinteren Teils (244) angeordnet sind, um durch den Daumen der linken Hand oder der rechten Hand gedrückt zu werden.

8. Gastrointestinaltraktdiagnosesystem nach Anspruch 6, wobei der Bauchabschnitt (243) und der Magnetstab (21) einen Winkel zwischen 155 und 175 Grad ausbilden.

9. Gastrointestinaltraktdiagnosesystem nach Anspruch 7, wobei der Bauchabschnitt (243) und der Magnetstab (21) einen Winkel zwischen 155 und 175 Grad ausbilden.

10. Gastrointestinaltraktdiagnosesystem nach Anspruch 7, wobei die erste Taste (C41) und die zweite Taste (C42) jeweils eine erste Taste des zweistufigen Drückens und eine zweite Taste eines zweistufigen Drückens sind.

11. Steuerungsverfahren für ein Gastrointestinaltraktdiagnosesystem, zumindest umfassend die folgenden Schritte:
(a) Bereitstellen eines Endoskops (10) mit einer Übertragungsleitung (160) und einer Magnetplatte (11), und Einbringen des Endoskops (10) in einen Hohlraumkörper (CV) mit einem ersten Abschnitt (160a) der Übertragungsleitung (160), wobei ein zweiter Abschnitt der Übertragungsleitung (160) außerhalb des Hohlraumkörpers (CV) verbleibt,
(b) Bereitstellen einer Magnetvorrichtung, die zumindest einen Magnetstab (21) und ein Magnetkraftausgabemodul (22) aufweist, und Aktivieren der Magnetvorrichtung, um dem Magnetkraftausgabemodul (22) zu ermöglichen, eine vorbestimmte magnetische Kraft zu erzeugen, magnetisch die Magnetplatte (11) anzuziehen, um somit den Hohlraumkörper (CV) durch Leiten des Endoskops (10) zu sichten,
(c) Bereitstellen eines Magnetkraftrückkopplungsmoduls (23) und Überwachen einer Rückkopplungsmagnetkraft, die durch die Magnetplatte (11) auf den Magnetstab (21) ausgeübt wird, wobei die Rückkopplungsmagnetkraft die vorbestimmte magnetische Kraft zu einer Offsetmagnetkraft redert, wobei das Magnetkraft-Rückkopplungsmodul (23) einen Kalibrierungsbefehl an das Magnetkraftausgabemodul (22) entsprechend der Rückkopplungsmagnetkraft aussendet, wodurch die Offsetmagnetkraft auf die vorbestimmte Magnetkraft zurückgeregelt werden kann,
(d) Einstellen von Bewegung, Rotation und dem Sichtfeld des Endoskops (10) innerhalb des Hohlraumkörpers (CV) mittels der Magnetvorrichtung, um Bilder einer Innenwand des Hohlraumkörpers (CV) aufzuzeichnen,
(e) Wiederholen der Schritte (b) bis zu dem Schritt (c), bis das Endoskop (10) Aufzeichnungsbilder der Innenwand des Hohlraumkörpers (CV) beendet,
(f) Ausschalten der magnetischen Vorrichtung, um die vorbestimmte magnetische Kraft abzustellen, und (g) herausziehen des zweiten Abschnitts der Übertragungsleitung (160), um das Endoskop (10) von der Innenseite des Hohlraumkörpers (CV) zu der Außenseite außerhalb des Hohlraumkörpers (CV) zu bewegen, um das Endoskop (10) zurückzuerhalten.

## Revendications

1. Système de diagnostic du tube digestif, comprenant au moins :
un endoscope (10) ayant une plaque magnétique (11) dans celui-ci, la plaque magnétique (11) ayant un premier pôle magnétique (111) et un second pôle magnétique (112) distribués respectivement dans une moitié inférieure et une moitié supérieure de la plaque magnétique (11) situées dans une direction d'épaisseur (TD) de la plaque magnétique (11) ; et
un dispositif de commande magnétique (20) comprenant :
une baguette magnétique (21) comprenant un premier pôle magnétique électrique (211) dans une partie terminale de la baguette magnétique (21) et un second pôle magnétique électrique (212) situé dans une partie embase de la baguette magnétique (21) ;
un module de production de force magnétique (22) destiné à être utilisé pour transmettre un courant prédéterminé à la baguette magnétique (21) de manière à permettre à la baguette magnétique (21) de produire un courant prédéterminé produisant une force magnétique prédéterminée pour interagir avec le premier pôle magnétique (111) et le second pôle magnétique (112) ; **caractérisé en ce que**
la force magnétique prédéterminée est apte à attirer la plaque magnétique (11) pour commander le déplacement de l'endoscope (10),
et la plaque magnétique (11) est apte à exercer une force magnétique de retour sur un capteur de la baguette magnétique (21), convertissant le courant prédéterminé en un courant de décalage, et **en ce que**
le dispositif de commande magnétique (20) comprend en outre un module de retour de force magnétique (23) dans lequel le module de retour de force magnétique (23) est apte à étalonner le courant de décalage, en ramenant le courant de décalage au courant prédéterminé, de manière à stabiliser la force magnétique prédéterminée.

2. Système de diagnostic du tube digestif selon la revendication 1, dans lequel l'endoscope (10) comprend :
un module de circuit (12) comprenant au moins une embase conductrice (121), un substrat (122) s'étendant à partir de l'embase conductrice (121) et une unité de commande (1220) placée sur le substrat (122) ;
un module de capture d'image (13) comprenant au moins une première lentille (131) et un capteur image (131a) placé sur le substrat (122) ;
un module d'éclairage (14) comprenant au moins une première source de lumière (141) placé sur le substrat (122) ;
un corps d'enveloppe (15) ; et
un dispositif de liaison filaire (16) ayant une interface conductrice (16a) et une ligne de transmission (160), une borne de la ligne de transmission (160) étant reliée à l'interface conductrice (16a) et l'autre borne de la ligne de transmission (160) étant reliée à un poste de travail (30),
dans lequel l'interface conductrice (16a) est reliée électriquement à l'unité de commande (1220) sur un côté de l'embase conductrice (121) ;
le corps d'enveloppe (15) est étanche et est assemblé au dispositif de liaison filaire (16) depuis l'autre côté de l'embase conductrice (121), enfermant le module de circuit (12), le module de capture d'image (13) et le module d'éclairage (14) ; et
la plaque magnétique (11) est fixée sur le module de circuit (12).

3. Système de diagnostic du tube digestif selon la revendication 2, dans lequel un support (1221) s'étend de manière inclinée à partir d'une extrémité libre du substrat (122), et la première lentille (131) est placée sur le support (1221).

4. Système de diagnostic du tube digestif selon la revendication 3, dans lequel le module de capture d'image (13) comprend une seconde lentille (132), la seconde lentille (132) étant placée sur une surface (122a) du substrat (122).

5. Système de diagnostic du tube digestif selon la revendication 4, dans lequel le corps d'enveloppe (15) comprend une première région polie (151), une seconde région polie (152) et une région d'uniformité matte (153) sur celui-ci, dans lequel la première région polie (151) correspond à la première lentille (131) et la seconde région polie (152) correspond à la seconde lentille (132).

6. Système de diagnostic du tube digestif selon la revendication 1, dans lequel le dispositif de commande magnétique (20) comprend :
une partie support (24) reliée à la partie embase de la baguette magnétique (21), la partie support (24) comprenant :
un premier côté (241) avec un premier bouton de démarrage (C1) préréglé sur un état inactif par défaut sur celui-ci ;
un second côté (242) avec un second bouton de démarrage (C2) préréglé sur un état inactif par défaut sur celui-ci ;
une partie ventrale (243) avec une bague (C3) sur celle-ci pour commander un état de rotation de la baguette magnétique (21) ; et
une partie dorsale (244) avec des boutons de manipulation (C4) et un écran (C5) sur celle-ci, dans lequel le premier bouton de démarrage (C1) et le second bouton de démarrage (C2) sont destinés à être actionnés en premier pour débloquer respectivement l'état inactif par défaut de la bague (C3) et des boutons de manipulation (C4).

7. Système de diagnostic du tube digestif selon la revendication 6, dans lequel les boutons de manipulation (C4) comprennent des boutons d'un menu (C43), une première touche (C41) et une seconde touche (C42),
dans lequel la première touche (C41) est agencée sur un premier emplacement (2441) de la partie dorsale (244), de manière à pouvoir être enfoncée par un pouce d'une main droite ;
la seconde touche (C42) est agencée sur un deuxième emplacement (2442) de la partie dorsale (244), de manière à pouvoir être enfoncée par un pouce d'une main gauche ; et
les buttons du menu (C43) sont agencés sur un troisième emplacement (2443) de la partie dorsale (244), de manière à pouvoir être enfoncés par le pouce de la main gauche ou de la main droite.

8. Système de diagnostic du tube digestif selon la revendication 6, dans lequel la partie ventrale (243) et la baguette magnétique (21) forment un angle compris entre 155 degrés et 175 degrés.

9. Système de diagnostic du tube digestif selon la revendication 7, dans lequel la partie ventrale (243) et la baguette magnétique (21) forment un angle compris entre 155 degrés et 175 degrés.

10. Système de diagnostic du tube digestif selon la revendication 7, dans lequel la première touche (C41) et la seconde touche (C42) sont une première touche d'un appui en deux temps et une deuxième touche d'un appui en deux temps, respectivement.

11. Procédé de commande d'un système de diagnostic du tube digestif, comprenant au moins les étapes suivantes :
(a) fournir un endoscope (10) ayant une ligne de transmission (160) et une plaque magnétique (11), et poser l'endoscope (10) dans un corps de cavité (CV) en laissant une première section (160a) de la ligne de transmission (160) et une seconde section de la ligne de transmission (160) à l'extérieur du corps de cavité (CV) ;
(b) fournir un dispositif magnétique ayant au moins une baguette magnétique (21) et un module de production de force magnétique (22), et activer le dispositif magnétique pour permettre au module de production de force magnétique (22) de produire une force magnétique prédéterminée pour attirer par voie électromagnétique la plaque magnétique (11), de manière à observer le corps de cavité (CV) en dirigeant l'endoscope (10) ;
(c) fournir un module de retour de force magnétique (23), et maintenir une surveillance d'une force magnétique de retour exercée par la plaque magnétique (11) sur la baguette magnétique (21), la force magnétique de retour transformant la force magnétique prédéterminée en une force magnétique de décalage, le module de retour de force magnétique (23) envoyant une commande d'étalonnage au module de production de force magnétique (22) en fonction de la force magnétique de retour, permettant de rectifier la force magnétique de décalage pour la ramener à la force magnétique prédéterminée
(d) régler le déplacement, la rotation et le champ visuel de l'endoscope (10) à l'intérieur du corps de cavité (CV) au moyen du dispositif magnétique pour enregistrer des images d'une paroi interne du corps de cavité (CV) ;
(e) répéter l'étape (b) à l'étape (c) jusqu'à ce que l'endoscope (10) ait fini d'enregistrer des images de la paroi interne du corps de cavité (CV) ;
(f) éteindre le dispositif magnétique pour mettre fin à la force magnétique prédéterminée ; et
(g) tirer sur la seconde section de la ligne de transmission (160) pour faire passer l'endoscope (10) de l'intérieur du corps de cavité (CV) à l'extérieur du corps de cavité (CV), de manière à récupérer l'endoscope (10).
